# EUROPÄISCHE PATENTANMELDUNG

(11) **EP 1 438 986 A2**
(43) Veröffentlichungstag der Anmeldung: **21.07.2004**
(21) Anmeldenummer: 04000239.6
(22) Anmeldetag: 08.01.2004
(51) Int. Cl.: A61N 1/39, A61B 5/00, A61N 1/362

(54) **Verfahren und Vorrichtung zur Erfassung einer Herzfunktionsstörung eines Patienten**

(30) Priorität: 11.01.2003 DE 10300735
(71) Anmelder: Corscience GmbH & CO.KG, 91052 Erlangen (DE)
(72) Erfinder: Bolz, Armin, Dr., 91054 Buckenhof (DE)
(74) Vertreter: Herrmann, Uwe

(57) **Zusammenfassung**

Das Verfahren und die Vorrichtung dienen zur Erfassung einer Störung der Herztätigkeit eines Patienten bzw. zur Durchführung einer Defibrillation. Von mindestens einem Sensor (12) wird mindestens ein die Herztätigkeit eines Patienten charakterisierender Parameter erfasst. Es wird eine automatische Auswertung im Hinblick auf mindestens einen die Störung der Herztätigkeit charakterisierenden Parameter durchgeführt und bei einer Erkennung einer Grenzwertüberschreitung für mindestens einen der die Störung charakterisierenden Parameter wird ein Alarmsignal generiert. Der Defibrillator (1) ist als eine mobile Einheit ausgebildet und mit einem Spannungsgenerator, (7) einer Steuereinheit (9) sowie mindestens zwei Elektroden (2,3) versehen.

## Beschreibung

Die Erfindung betrifft ein Verfahren und eine Vorrichtung zur Erfassung einer Störung der Herztätigkeit eines Patienten.

Ein zunehmender Anteil älterer Mitmenschen in unserer Gesellschaft führt zu einer signifikant steigenden Zahl kardiopulmonaler Erkrankungen mit zum Teil lebensbedrohlichen Folgen. An erster Stelle zu nennen ist dabei der Herzinfarkt, der in der Folge zu einer Schädigung des Myokards und schließlich zu einer Herzinsuffizienz (CHF: Congestive Heart Failure) führen kann. Die daraus resultierenden Kreislaufveränderungen haben häufig auch Auswirkungen auf die pulmonalen Parameter, so dass diese Patienten nicht selten zusätzlich beatmungspflichtig werden. Darüber hinaus gehört diese Gruppe zu den Risikopatienten, da das vorgeschädigte Herz zu Kammerflimmern neigt, was schließlich zum plötzlichen Herztod führt.

Für alle Teilbereiche der betreffenden Erkrankungen gibt es mittlerweile Therapieverfahren. Die Atemschwierigkeiten lassen sich wirkungsvoll durch CPAP-Geräte oder daraus abgeleitete Verfahren behandeln, ein Kammerflimmern (VP) kann durch Defibrillation behandelt werden. Medikamentöse Therapien befinden sich weitgehend auf dem Rückmarsch, da in umfangreichen Studien lediglich kurzfristige Erfolge nachweisbar waren, langfristig jedoch proarrhythmische Effekte vorherrschen.

Während die CPAP-Therapie aufgrund der vergleichsweise geringen Kosten und der einfacheren Handhabbarkeit auch im Heimbereich angewandt und vor allem durch die Krankenkassen bezahlt wird, findet die Defibrillation erst langsam Akzeptanz. Implantierbare Defibriiiatoren (sog. ICDs) sind mit einem Gerätepreis von etwa 20.000 Euro zu teuer und werden daher unter anderem erst nach einem überlebten VF-Anfall von den Krankenkassen genehmigt. Mehr und mehr setzen sich externe Defibrillatoren im präklinischen Bereich durch. Aufgrund der langen Anfahrtzeiten der Notarztfahrzeuge kommt die externe Hilfe jedoch in aller Regel zu spät. Derzeit überleben in Deutschland nur etwa 2% aller VF-Patienten ihren ersten Anfall. Daraus ergibt sich die dringende Notwendigkeit, nach neuen, kostengünstigeren und zugleich einfacheren Methoden zu suchen, die eine höhere Überlebensrate in dieser umfangreichen Patientengruppe sicherstellen.

Seit wenigen Jahren werden unter dem Begriff AED(Automatic External-Defibrillator) bzw. PAD (Public Access Defibrillator) sogenannte halbautomatische Defibrillatoren eingeführt, die dank ihrer automatischen Diagnosefunktion eine Bedienung durch medizinische Laien ermöglichen. Hierdurch wird - bei flächendeckender Verbreitung im Sinne eines Feuerlöschers - die Anfahrtzeit des Notarztes vermieden und somit die Überlebenswahrscheinlichkeit erhöht. Eine umfangreiche Studie auf dem Flughafen Chicago O'Hare hat beispielsweise ergeben, dass sich die Rate der erfolgreichen Reanimationen auf bis zu 90 % anheben läßt, sofern das Flughafengebäude mit derartigen Geräten ausgestattet und das Personal in deren Handhabung eingewiesen wird. Auf diese Weise ließ sich allein im Jahre 2000 eine Anzahl von 12 Patienten auf diesem Flughafen erfolgreich reanimieren. Speziell für Risikopatienten bietet sich somit die Möglichkeit, derartige Geräte zu erwerben und zu Hause für Familienangehörige zugänglich aufzubewahren oder aber mit sich zu führen.

Einen anderen Ansatz verfolgen seit kurzem die Firmen Medtronic und Lifecor. Sie entwickelten eine tragbare Weste, in die ein Defibrillator eingearbeitet ist. Er analysiert das EKG des Patienten und aktiviert im Ernstfall vollautomatisch die ebenfalls in die Weste eingenähten Elektroden, drückt pneumatisch Elektrodengel an die Grenzfläche der Elektrode-Haut und defibrilliert. Die kürzlich veröffentlichte FDA-Studie zeigte, dass die Überlebensrate auf 25 % ansteigt. Dieses Ergebnis zeigt, dass tragbare externe Defibrillatoren eine sinnvolle Alternative zu bestehenden Konzepten bieten.

Zu beachten ist aber, dass trotzdem ein Teil der Patienten ihren Anfall nicht überlebt, wenn sie zum entsprechenden Zeitpunkt das Gerät nicht angelegt haben. Insbesondere in der Nacht ist ein derartiges Gerät nicht akzeptabel, da es aufgrund der externen Komponenten keinen erholsamen Schlaf zuläßt.

Hinzu kommt, dass ein derartiges Gerät wegen der technisch aufwendigen Konstruktion immer noch in einem Preisbereich von 10.000 Euro liegt und somit auch ökonomisch keine wesentlichen Vorteile bietet. Der einzige Vorteil besteht in der Tatsache, dass externe Geräte als Investitionsgut angeschafft werden können und sich damit von Patient auf Patient übertragen lassen, Implantate dagegen als Verbrauchsgut behandelt werden und somit in das Eigentum eines Patienten übergehen. Eine Übertragung, bspw. im Sinne eines Bridging Devices zwischen Transplantationskandidaten, ist somit rechtlich bedenklich.

Stand der Technik ist somit eine Therapie durch externe Defibrillation in allen Lebenslagen, in denen der Patient unter Überwachung steht. Insbesondere bei Nacht sowie bei Alleinstehenden bzw. außerhalb von klinisch ausgerüsteten Überwachungseinrichtungen (Kliniken, Pflegeheimen etc.) bietet dieser Stand der Technik jedoch keinen ausreichenden Schutz.

Aufgabe der vorliegenden Erfindung ist es, ein Verfahren der einleitend genannten Art derart zu verbessern, dass eine Geräteaktivierung mit geringer Zeitverzögerung unterstützt wird.

Diese Aufgabe wird erfindungsgemäß dadurch gelöst, dass von mindestens einem Sensor mindestens ein die Herztätigkeit eines Patienten charakterisierender Parameter erfaßt wird, dass eine automatische Auswertung im Hinblick auf mindestens einen die Störung der Herztätigkeit des Patienten charakterisierenden Parameter durchgeführt wird und dass bei einer Erkennung einer Grenzwertüberschreitung für mindestens einen die Störung der Herztätigkeit charakterisierenden Parameter ein Alarmsignal generiert wird.

Der Begriff "Grenzwertüberschreitung" ist allgemein zu verstehen und umfasst beispielsweise auch eine auf der Fuzzy-Logik basierende Auslösung des Alarms. Dabei kann beispielsweise ein Alarmsignal generiert werden, wenn Parameter erfasst werden, die einzeln noch nicht als kritisch bewertet werden, in ihrer Summe jedoch einen kritischen Zustand charakterisieren, der eine Alarmauslösung bedingt.

Bei der Störung der Herztätigkeit eines Patienten kann es sich um eine beliebige Abweichung der Herztätigkeit vom Normalzustand handeln. Zu diesen Störungen sind beispielsweise das Kammerflimmern, das Kammerflattern, die Kammertachykardie sowie die Asystolie zu zählen. Auch beliebige andere Störungen der Herzaktivität kommen in Betracht.

Bei dem Alarmsignal kann es sich beispielsweise um ein Signal handeln, das einen Defibrillator aktiviert und gleichzeitig oder zeitversetzt beispielsweise akustisch oder optisch wahrnehmbar ist. Des Weiteren ist von dem Begriff "Alarmsignal" auch ein Signal umfasst, das nicht zu einer unmittelbaren Aktivierung eines Defibrillators führt, sondern lediglich im Sinne einer Statusinformation darüber informiert, dass eine Grenzwertüberschreitung eingetreten ist. Ein derartiges Signal kann in beliebiger Weise, z.B. optisch oder akustisch abgegeben werden.

In bevorzugter Ausgestaltung der Erfindung ist vorgesehen, dass die Signalerfassung am Patienten und die Generierung eines Alarmsignals von diesem örtlich getrennt durchgeführt werden. Dabei kann vorgesehen sein, dass die Signalerfassung mittels mobiler, tragbarer Sensoren erfolgt. Die Generierung eines Alarmsignals kann beispielsweise in unmittelbarer Nähe des Patienten, beispielsweise auf dem Nachttisch, in einem Arzt- oder Stationszimmer, bei einer Rettungsleitstelle, bei Angehörigen oder Freunden etc. erfolgen.

Die Signalauswertung kann am Patienten oder von diesem örtlich getrennt angeordnet sein. Die Datenübertragung zwischen dem oder den Sensoren und der Signalauswertung sowie zwischen der Signalauswertung und den Mitteln zur Generierung eines Alarmsignals kann auf beliebige Weise, beispielsweise schnurgebunden oder drahtlos erfolgen.

Weitere vorteilhafte Ausgestaltungen des erfindungsgemäßen Verfahrens sind Gegenstand der Unteransprüche.

Bei der Störung der Herztätigkeit eines Patienten kann es sich beispielsweise um einen Fibrillationszustand und bei dem die Störung der Herztätigkeit charakterisierenden Parameter um einen Fibrillationsparameter handeln.

Eine aussagefähige Erfassung der jeweiligen Herztätigkeit kann dadurch erfolgen, dass eine messtechnische Erfassung eines EKG-Signals durchgeführt wird. Auch eine Erfassung eines Puls-Signals oder eines Hämodynamik-Signals ist alternativ oder zusätzlich durchführbar.

Ein großer Anwendungskomfort kann dadurch unterstützt werden, dass die Messwerterfassung im Bereich mindestens eines Klebepflasters durchgeführt wird. Ebenfalls ist daran gedacht, dass die Messwerterfassung im Bereich mindestens eines Armbandes, Halsbandes, Bauchbandes oder Hüftbandes durchgeführt wird.

Eine Signalerfassung in unmittelbarer Herznähe wird dadurch unterstützt, dass die Messwerterfassung im Bereich eines Brustbandes durchgeführt wird.

Ein weiter gesteigerter Benutzungskomfort kann dadurch erreicht werden, dass eine sensorische Messdatenerfassung und eine Auswertung der Messsignale voneinander örtlich getrennt durchgeführt werden.

Insbesondere ist daran gedacht, dass die vom Sensor erfaßten Messdaten drahtlos zu einer Signalauswertung übertragen werden. In diesem Fall trägt der Patient im wesentlichen nur die Sensoren sowie Mittel zur Datenübertragung und eine Stromversorgung mit sich, während die Signalauswertung sowie die Mittel zur Generierung von Alarmsignalen davon entfernt, beispielsweise ortsfest oder auch mobil ausgeführt sein können.

Ebenso ist denkbar, dass eine sensorische Messdatenerfassung und eine Auswertung der Messsignale örtlich benachbart durchgeführt werden und dass die Ergebnisse der Signalauswertung übertragen werden.

Eine kompakte Ausführungsform wird dadurch erreicht, dass eine Messdatenerfassung im Bereich einer Beatmungsmaske durchgeführt wird.

Eine typische Signalgenerierung erfolgt derart, dass ein akustisches Alarmsignal generiert wird. Darüber hinaus ist auch daran gedacht, dass ein optisches Alarmsignal generiert wird.

Besonders vorteilhaft ist es, wenn die Werte des mindestens einen die Herztätigkeit eines Patienten charakterisierenden Parameters gespeichert werden. Die Speicherung kann an beliebiger Stelle erfolgen. Denkbar ist beispielsweise, dass die Daten in einem Modul gespeichert werden, das der Patient mit sich trägt. Ebenso ist es möglich, die Speicherung in einem stationär angeordneten Speicher, beispielsweise in einem Server vorzunehmen. Die abrufbare Speicherung dieser Daten bringt den Vorteil mit sich, dass der Krankheitsverlauf bzw. der Zustand des Patienten vor Eintritt eines Notfalls verfolgt werden kann, wodurch eine Entscheidung hinsichtlich der weiteren vorzunehmenden Maßnahmen erleichtert werden kann. Die Werte können in dem Speicher abrufbar vorliegen. Es kann ebenso vorgesehen sein, dass die Speicher die Werte aktiv versenden.

Die Werte des mindestens einen die Herztätigkeit eines Patienten charakterisierenden Parameters können unmittelbar von dem Sensor an den Speicher übertragen werden. Ebenso ist es denkbar, dass die Werte zunächst vom Sensor an eine Signalauswertung übertragen werden und von dieser dem Speicher zugeführt werden. Dabei kann die Übertragung von der Signalauswertung an den Speicher beispielsweise schnurlos erfolgen.

In bevorzugter Ausgestaltung der Erfindung ist vorgesehen, dass bei Erkennen einer Grenzwertüberschreitung ein Kennzeichnungssignal generiert wird, das die Abgabe des Alarmsignals bewirkt. Das Kennzeichnungssignal kann beispielsweise von einem Signalgenerator erzeugt werden und bewirken, dass ein Signalgeber das Alarmsignal abgibt.

Das Kennzeichnungssignal kann schnurgebunden oder drahtlos übertragen werden. Hinsichtlich der Übertragungstechnik bestehen keine Beschränkungen. Die Übertragung des Kennzeichnungssignals kann per Datennahübertragung, insbesondere per Bluetooth, oder per Datenfernübertragung, insbesondere mittels Telefon oder Mobilfunktechnik oder per Funk, erfolgen.

Soll die Datennahübertragung angewandt werden, kann das Alarmsignal von einem im Empfangsbereich des Kennzeichnungssignals befindlichen Signalgeber beispielsweise optisch und/oder akustisch abgegeben werden. Selbstverständlich ist es ebenso möglich, dass die Versorgung des Signalgebers mit dem Kennzeichnungssignal schnurgebunden erfolgt.

Kommt die Datenfernübertragung zum Einsatz, besteht die Möglichkeit, das Kennzeichnungssignal an ein mobiles Endgerät, wie beispielsweise an ein Funkgerät, ein Handy oder PDA zu übertragen, das bei Empfang des Kennzeichnungssignals das Alarmsignal abgibt. Auf diese Weise ist es möglich, dass ein Alarmsignal an einem beliebigen Ort und losgelöst vom Aufenthaltsort des Patienten abgegeben wird. Als Empfänger des Alarmsignals kommen beispielsweise Angehörige, der Notarzt bzw. eine Rettungsleitstelle in Betracht. Beispielsweise kann in der Wohnung des Patienten oder von Angehörigen ein lokaler Melder aufgestellt sein, der das Alarmsignal abgibt.

Denkbar ist es beispielsweise, dass das Alarmsignal in Form eines Anrufes mit einer Sprachnachricht oder in Form einer SMS übermittelt wird.

Weiterhin kann vorgesehen sein, dass im Rahmen der Übertragung des Kennzeichnungssignals die gespeicherten Werte des mindestens einen die Herztätigkeit eines Patienten charakterisierenden Parameters oder Informationen über einen Speicherplatz übertragen werden, an dem die Werte abrufbar vorliegen. Die Übertragung dieser Informationen kann zeitgleich oder auch zeitversetzt zur der Übertragung des Kennzeichnungssignals oder auch davon unabhängig erfolgen. Eine derartige Ausgestaltung der Erfindung ermöglicht es, den Empfänger nicht nur mit dem Alarmsignal, sondern auch mit Informationen über den Zustand des Patienten vorzugsweise über einen längeren Zeitraum, insbesondere über den Zustand des Patienten vor Eintritt eines Notfalls zu versorgen, wodurch dem behandelnden Arzt eine Entscheidungshilfe hinsichtlich der weiteren Behandlungsschritte an die Hand gegeben werden kann.

Denkbar ist beispielsweise, den das Alarmsignal erhaltenden Arzt mit den Werten der die Herztätigkeit eines Patienten charakterisierender Parameter zu versorgen, die beispielsweise auf einem PDA sofort visualisiert werden können. In Betracht kommt beispielsweise die Darstellung des EKG auf dem PDA.

Die Werte können vor, während oder im Anschluss an die Übersendung des Kennzeichnungssignals übertragen werden. Entsprechendes gilt für die Information hinsichtlich des Speicherplatzes, die beispielsweise implizit in dem Alarmsignal enthalten sein kann. Beispielsweise ist denkbar, dass das Alarmsignal aus dem Namen des Patienten oder einer Gerätenummer eines Defibrillators besteht und dass unter diesen Bezeichnungen auch die gespeicherten Daten zu finden sind. Diese können beispielsweise auf einem Server abgelegt sein.

Entsprechendes gilt in weiterer Ausgestaltung der Erfindung auch für Patientendaten oder Informationen über einen Speicherplatz, an dem Patientendaten abrufbar vorliegen. Hierbei ist vorgesehen, dass der Adressat des Alarmsignals auf wichtige Patientendaten zugreifen kann, um eine optimale weitere Behandlung des Patienten sicherzustellen. Derartige Patientendaten können Daten zu bisherigen Krankheiten, zu eingenommenen Medikamenten, zu Medikamentenunverträglichkeiten etc. umfassen.

Die Werte des die Herztätigkeit eines Patienten charakterisierendes Signals und/oder die Patientendaten können beispielsweise mittels Download geladen werden oder per Internet-Browser eingesehen werden.

In weiterer Ausgestaltung der Erfindung kann vorgesehen sein, dass der Empfänger des Alarmsignals die Möglichkeit hat, mittels schnurloser Datenübertragung eine Aktivierung eines Defibrillators vorzunehmen. Denkbar ist der Einsatz der Mobilfunktechnik.

Im weiterer Ausgestaltung der Erfindung ist vorgesehen, dass erfasst wird, ob und wie sich der Patient bewegt, und dass diese Informationen zusammen mit den die Herztätigkeit eines Patienten charakterisierenden Parametern zur Bestimmung einer Grenzwertüberschreitung herangezogen werden. Auf diese Weise kann eine besonders genaue Ermittlung einer Grenzwertüberschreitung vorgenommen werden, da die Grenzwertberechnung die Bewegungen des Patienten berücksichtigt.

Weitere Aufgabe der vorliegenden Erfindung ist es, eine Vorrichtung der einleitend genannten Art derart zu konstruieren, dass eine verbesserte Einsatzfähigkeit unterstützt wird.

Diese Aufgabe wird erfindungsgemäß durch eine Vorrichtung zur Erfassung einer Störung der Herztätigkeit eines Patienten mit mindestens einem Sensor zur Erfassung mindestens eines eine Herztätigkeit eines Patienten charakterisierenden Signals, mit wenigstens einer Signalauswertung, an die der Sensor angeschlossen ist, wobei die Signalauswertung mit einem Analysator zur Erkennung einer Grenzwertüberschreitung für mindestens einen die Störung der Herztätigkeit charakterisierenden Parameter versehen ist, sowie mit einem Signalgeber, an den die Signalauswertung angeschlossen ist, gelöst.

Bei der Störung der Herztätigkeit eines Patienten kann es sich um eine beliebige Störung, beispielsweise um Rhythmusstörungen, oder einen sonstigen krankhaften Zustand handeln. Beispielsweise handelt es sich bei der Störung der Herztätigkeit eines Patienten um einen Fibrillationszustand und bei dem die Störung charakterisierenden Parameter um einen Fibrillationsparameter.

In einer bevorzugten Ausführungsform der Erfindung ist vorgesehen, dass der oder die Sensoren als tragbare, mobile Sensoren ausgeführt sind. Die Signalauswertung kann ebenfalls an dem Patienten angeordnet oder auch an anderer Stelle, z. B. ortsfest ausgeführt sein. In bevorzugter Ausgestaltung der Erfindung ist der Signalgeber örtlich vom Patienten getrennt, also nicht unmittelbar am Patienten angeordnet. Dementsprechend kann vorgesehen sein, dass der oder die Sensoren und ggf. die Signalauswertung am Patienten getragen werden, wohingegen dies für den Signalgeber in bevorzugter Ausgestaltung der Erfindung nicht gilt. Der Signalgeber kann sich dennoch in der Nähe des Patienten, beispielsweise am Bett oder in der Nähe des Bettes, oder auch in größerer Entfernung vom Patienten, beispielsweise in einem Arztzimmer, einer Rettungsleitstelle, bei Freunden oder Verwandten befinden.

Weitere vorteilhafte Ausgestaltungen der Vorrichtung sind Gegenstand der Unteransprüche.

In weiterer Ausgestaltung der Erfindung ist vorgesehen, dass der Signalgeber von einem Signalgenerator aktivierbar ist.

Es kann mindestens ein Sensor zur Erfassung mindestens eines eine Herztätigkeit eines Patienten charakterisierenden Signals an die Steuereinheit angeschlossen sein, wobei der Sensor mit einer Auswertungseinheit zur Erkennung einer Grenzwertüberschreitung für mindestens einen Fibrillationsparameter verbunden ist und wobei die Auswertungseinheit an einen Signalgeber angeschlossen ist, der von einem Signalgenerator aktivierbar ist.

In weiterer Ausgestaltung der Erfindung handelt es sich bei der erfindungsgemäßen Vorrichtung zur Erfassung einer Störung der Herztätigkeit eines Patienten um eine Vorrichtung zur Defibrillation, die als eine mobile Einheit ausgebildet und mit einem Spannungsgenerator, einer Steuereinheit sowie mindestens zwei Elektroden versehen ist, wobei mindestens ein Sensor zur Erfassung mindestens eines eine Herztätigkeit eines Patienten charakterisierendes Signals an eine Signalauswertung angeschlossen ist, wobei die Signalauswertung mit einem Analysator zur Erkennung einer Grenzwertüberschreitung für mindestens einen Fibrillationsparameter versehen ist und wobei die Signalauswertung an einen Signalgeber angeschlossen ist, der von einem Signalgenerator aktivierbar ist.

Durch die Verwendung und Auswertung eines Sensors zur Erfassung mindestens eines Parameters, der die Herztätigkeit des Patienten charakterisiert und durch die gerätetechnische Auswertung der Messwerte im Hinblick auf eine Grenzwertüberschreitung für den mindestens einen Parameter, beispielsweise einen Fibrillationsparameter, ist es möglich, messtechnisch rechtzeitig einen Zeitpunkt für eine erforderliche Geräteaktivierung zu erkennen und über den zugeordneten Signalgenerator und den Signalgeber die erforderlichen Steuersignale zu generieren. Eine Aktivierung des Defibrillators kann entweder unmittelbar durch die vom Signalgeber erzeugten Signale erfolgen, ebenfalls ist daran gedacht, durch den Signalgeber unmittelbar lediglich ein Statussignal abzugeben, das über eine nachgeschaltete Signalauswertung zu einer Aktivierung des Defibrillators herangezogen wird.

Die Sensoren überwachen vorzugsweise kontinuierlich die eine Herztätigkeit eines Patienten charakterisierenden Signale. Die Auswerteeinheit umfasst in bevorzugter Ausgestaltung der Erfindung digitale Signalprozessoren.

Eine kompakte Geräteausbildung wird auch dadurch unterstützt, dass die Signalauswertung als Teil der Steuereinheit ausgebildet ist.

Eine hohe Aufstellungsflexibilität kann dadurch erreicht werden, dass die Signalauswertung örtlich getrennt zur Steuereinheit angeordnet ist.

Der Sensor der erfindungsgemäßen Vorrichtung kann zur Erfassung mindestens eines EKG-Signals, eines Puls-Signals und/oder eines Hämodynamiksignals ausgebildet sein.

Der Sensor kann im Bereich mindestens eines Klebepflasters, Armbandes, Halsbandes, Brustbandes, Bauchbandes, Hüftbandes sowie im Bereich einer Atemmaske angeordnet sein. Denkbar ist beispielsweise, dass mehrere Klebepflaster und/oder mehrere Armbänder mit entsprechenden Sensoren vorgesehen sind.

Der Sensor kann benachbart zur Signalauswertung oder örtlich getrennt zur Signalauswertung angeordnet sein. In weiterer Ausgestaltung der Erfindung ist der Sensor drahtlos mit der Signalauswertung gekoppelt. Dabei kann vorgesehen sein, dass der Patient nur den oder die Sensoren mit sich trägt und dass deren Signale an die Signalauswertung übertragen werden. Auch ist es möglich, dass der Patient den oder die Sensoren sowie die Signalauswertung mit sich führt und dass das Ergebnis der Signalauswertung sodann beispielsweise drahtlos an einen Signalgenerator übertragen wird, was den Vorteil mit sich bringt, dass eine Datenübertragung bevorzugt nur dann stattfinden muss, wenn die Signalauswertung eine Grenzwertüberschreitung ergibt.

Denkbar ist somit eine Ausführungsform, bei der der Patient lediglich einen oder mehrere mobile tragbare Sensoren, eine Datenübermittlungseinheit und eine Stromversorgung mit sich führt, während die weiteren Komponenten der Vorrichtung davon örtlich getrennt sind. Die Generierung des Alarmsignals kann ein geeigneter Stelle, beispielsweise in einem Arztzimmer, der Rettungsleitstelle, bei Angehörigen oder auch in der Nähe des Patienten, beispielsweise auf dem Nachttisch erfolgen.

Der Signalgeber kann beliebig ausgeführt sein. In Betracht kommt beispielsweise die Ausführung als akustischer oder optischer Signalgeber.

In bevorzugter Ausgestaltung der Erfindung ist ein Speicher vorgesehen, in dem die Werte des mindestens einen die Herztätigkeit eines Patienten charakterisierenden Parameters und/oder Patientendaten gespeichert werden. Der Speicher kann mobil ausgeführt sein, d.h. am Patienten getragen werden. Der Speicher kann jedoch auch stationär angeordnet sein, beispielsweise in Form eines Servers. Es kann vorgesehen sein, dass der oder die Sensoren die erfassten Daten kontinuierlich dem Speicher zuführen. Die Übertragung der Daten kann schnurgebunden oder drahtlos erfolgen. Vorteilhaft ist es, die Daten für einen vorgegebenen Zeitraum (Z. B. über mehrere Wochen oder Monate) aufzuzeichnen, damit dem medizinischen Personal eine Bewertung des Krankheitsverlaufes bzw. der Entstehung des aktuellen Zustandes ermöglicht wird.

Es können Mittel vorgesehen sein, durch die ein von dem Signalgenerator abgegebenes Kennzeichnungssignal schnurgebunden oder drahtlos übertragbar ist. In Betracht kommen beliebige Übertragungstechniken, wie beispielsweise Bluetooth oder die Mobilfunktechnik.

Auch ist es denkbar, die Übertragung der Werte der die Herztätigkeit des Patienten charakterisierenden Parameter in Echtzeit vornehmen zu können, was für eine Funktionsüberprüfung vorteilhaft sein kann. Die Übertragung dieser Werte kann von dem oder den Sensoren selbst oder von einer daran angeschlossenen Auswerteeinheit vorgenommen werden.

Des Weiteren können Bewegungssensoren vorgesehen sein, mittels derer erfasst wird, ob und wie sich der Patient bewegt. Die von diesen Sensoren erfassten Signale können zusätzlich zu den erfassten Parametern zu Feststellung herangezogen werden, ob eine Grenzwertüberschreitung vorliegt.

Grundsätzlich ist es möglich, dass die Sensoren zur Erfassung des die Herztätigkeit des Patienten charakterisierenden Parameters durch Defibrillatorelektroden gebildet werden. Auch ist es möglich, zusätzlich zu den Defibrillatorelektroden Sensoren zur Erfassung der die Herztätigkeit charakterisierenden Parameter vorzusehen.

Es können Mittel vorgesehen sein, anhand derer Informationen zu dem Aufenthaltsort des Patienten erhältlich sind, um eine rasche Hilfestellung leisten zu können. Diese Informationen können beispielsweise Bestandteil des von dem Signalgenerator erzeugten Kennzeichnungssignals sein und sodann vom Signalgeber an dem Empfänger des Alarmsignals übergeben werden.

Denkbar ist, dass die erfindungsgemäße Vorrichtung permanent oder in bestimmten Zeitabständen Informationen zum aktuellen Aufenthaltsort der Vorrichtung und somit auch des Patienten erhält, diese speichert und im Bedarfsfall übersendet. Eine derartige Empfangs- und Speichereinheit kann beispielsweise in die Signalauswertung integriert sein. Derartige Informationen können beispielsweise Ortsinformationen, wie z.B. Koordinaten oder Kennungen von Funkzellen oder Location Areas eines Mobilfunknetzes darstellen, die von einer Sendeeinheit des Netzes zur Verfügung gestellt werden. In diesem Fall ist die erfindungsgemäße Vorrichtung mit Mitteln ausgeführt, die die Erfassung dieser Ortsdaten ermöglichen. Auch andere aus dem Bereich der Navigationssysteme bekannte Lokalisierungstechniken sind anwendbar. Eine genaue Standortbestimmung ist z.B. per GPS realisierbar, wozu die erfindungsgemäße Vorrichtung mit einem entsprechenden Empfänger zu versehen ist. Die Standortdaten können gespeichert und im Bedarfsfall an einen Adressaten übersandt werden oder abgefragt werden.

In weiterer Ausgestaltung der Erfindung sind Mittel vorgesehen, die kontinuierlich oder in Zeitabständen einen Selbsttest der Vorrichtung durchführen.

Die Sensoren, die Elektroden und/oder die Auswerteeinheit können vorzugsweise in Kleidungsstücke (BHs, Slips, Gürtel etc.) integriert sein. Es kann vorgesehen sein, dass insbesondere die Sensoren und/oder die Elektroden derart ausgeführt sind, dass diese beim Waschen der Kleidungsstücke nicht entfernt werden müssen. Die Auswerteeinheit und etwaige weitere Komponenten der Vorrichtung sind vorzugsweise lösbar von dem Kleidungsstück ausgeführt.

Desweiteren kann vorgesehen sein, dass die Stromversorgung für die Komponenten der Vorrichtung, insbesondere der am Patienten befindlichen Komponenten mittels einer wiederaufladbaren Batterie erfolgt.

In den Zeichnungen sind Ausführungsbeispiele der Erfindung schematisch dargestellt. Es zeigen:
Fig. 1: ein schematisches Blockschaltbild zur Veranschaulichung eines grundsätzlichen Aufbaus eines Defibrillators mit Überwachungseinrichtung,
Fig. 2: ein Blockschaltbild zur Veranschaulichung des grundsätzlichen Aufbaus der Überwachungseinrichtung,
Fig. 3: eine gegenüber Fig. 2 abgewandelte Ausführungsform, bei der ein Sensor und eine Signalauswertung räumlich getrennt voneinander angeordnet und drahtlos miteinander verbunden sind und
Fig. 4: eine nochmals abgewandelte Ausführungsform, bei der die Signalauswertung räumlich benachbart zum Sensor angeordnet ist und eine drahtlose Übertragung bereits vorverarbeiteter Daten erfolgt.

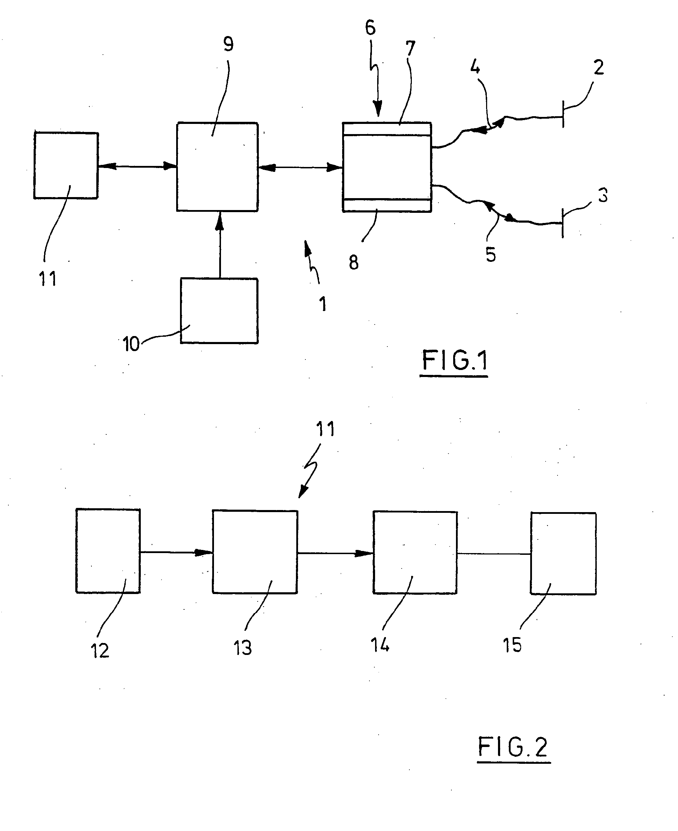

Figur 1 zeigt den prinzipiellen Aufbau eines Defibrillators (1). Der Defibrillator (1) weist zwei Elektroden (2, 3) auf, die über Anschlußleitungen (4, 5) an eine Basiseinheit (6) angeschlossen sind. Die Basiseinheit (6) ist mit einem Schockgeber (7) sowie einer Detektionseinheit (8) versehen.

Der Schockgeber (7) ist dazu ausgebildet, Elektroschocks an die Elektroden (2, 3) anzulegen. Die Detektionseinheit (6) ist insbesondere dafür ausgebildet, elektrische Signale im Bereich der Elektroden (2, 3) aufzunehmen, beispielsweise ein Elektrokardiogramm. Die Detektionseinheit (8) unterstützt es insbesondere, den Elektroschock zeitoptimal abzugeben.

Die Basiseinheit (6) ist an eine Steuereinheit (9) angeschlossen, die eine Auswertung der Signale der Detektionseinheit (8) vornehmen kann. Ebenfalls ist auch daran gedacht, dass die Detektionseinheit (8) unmittelbar auf den Schockgeber (7) einwirkt. Eine manuelle Betätigung der Steuereinheit kann über eine Bedieneinheit (10) erfolgen.

An die Steuereinheit (9) ist ebenfalls eine Überwachungseinrichtung (11) angeschlossen, die mindestens einen die Herztätigkeit des Patienten charakterisierenden Parameter erfaßt. Insbesondere ist daran gedacht, die Überwachungseinrichtung (11) als eine mobile EKG-Erfassungseinheit auszubilden. Die Überwachungseinrichtung (11) kann beispielsweise im Bereich eines Klebepflasters, im Bereich eines Armbandes oder im Bereich eines Brustbandes positioniert werden.

Figur 2 zeigt den Aufbau der Überwachungseinrichtung (11) in einer etwas detaillierteren Darstellung. Die Überwachungseinrichtung (11) besteht im wesentlichen aus einem Sensor (12), einer Signalauswertung (13), einem Signalgenerator (14) sowie einem Signalgeber (15). Im Bereich der Signalauswertung (13) erfolgt ein Vergleich, ob die gemessenen Parameter einen Fibrillationszustand charakterisieren.

Für den Fall einer Erkennung eines derartigen Zustandes wird über den Signalgenerator (14) ein geeignetes Kennzeichnungssignal generiert und über den Signalgeber (15) abgegeben. Der Signalgeber (15) kann entweder direkt an die Steuereinheit (9) angeschlossen sein, es ist aber auch möglich, dass das vom Signalgeber (15) abgegebene Signal lediglich mittelbar für eine Aktivierung des Defibrillators (1) verwendet wird.

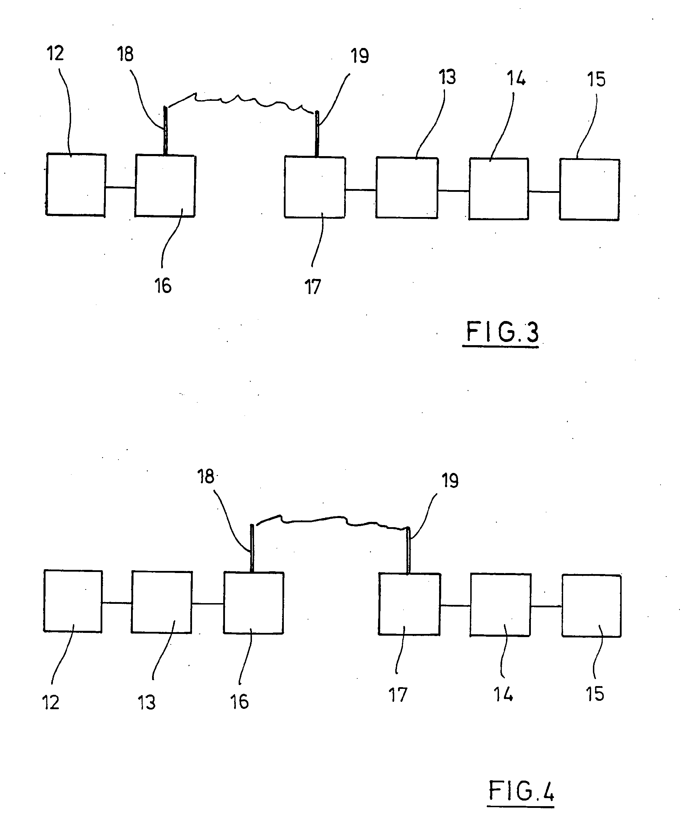

Gemäß der Ausführungsform in Figur 3 ist der Sensor (12) räumlich getrennt von der Signalauswertung (13) sowie den nachfolgenden Bauelementen angeordnet. Der Sensor (12) ist lediglich mit einem Sender (16) gekoppelt, der über eine Antenne (18) drahtlos mit einem Empfänger (17) gekoppelt ist, der eine Antenne (19) aufweist. Eine derartige Ausführungsform unterstützt eine leichte Ausbildung einer den Sensor (12) aufweisenden Erfassungseinheit und damit einen angenehmen Benutzungskomfort für den Patienten.

Gemäß einer besonderen Ausführungsform ist insbesondere daran gedacht, den Signalgeber (15) als einen akustischen Signalgeber oder einen optischen Signalgeber zu realisieren. Das derart generierte Alarmsignal kann erfaßt und für eine Aktivierung des Defibrillators (1) benutzt werden.

Gemäß einer weiteren Ausführungsform ist auch daran gedacht, die Detektionseinheit (8) mindestens zu einem Teil im Bereich einer Beatmungsmaske für die Durchführung der CPAP-Therapie anzuordnen. Hierdurch wird einem Patienten das Anlegen der Überwachungseinrichtung (11) erleichtert, da kein zusätzliches separates Teil zu benutzen ist.

Bei der Ausführungsform gemäß Figur 4 ist die Signalauswertung (13) örtlich benachbart zum Sensor (12) angeordnet. Durch diese Ausführungsformen kann der Umfang der vom Sender (16) zum Empfänger (17) zu übertragenden Daten reduziert werden. Dies führt beim Sender (16) zu einem geringeren Stromverbrauch und deshalb bei einem Batteriebetrieb zu einer verlängerten Betriebsfähigkeit.

## Patentansprüche

1. Verfahren zur Erfassung einer Störung der Herztätigkeit eines Patienten, **dadurch gekennzeichnet, dass** von mindestens einem Sensor (12) mindestens ein die Herztätigkeit eines Patienten charakterisierender Parameter erfaßt wird, dass eine automatische Auswertung im Hinblick auf mindestens einen die Störung der Herztätigkeit charakterisierenden Parameter durchgeführt wird und dass bei einer Erfassung einer Grenzwertüberschreitung für mindestens einen der die Störung der Herztätigkeit charakterisierenden Parameter ein Alarmsignal generiert wird.

2. Verfahren nach Anspruch 1, **dadurch gekennzeichnet, dass** es sich bei der Störung der Herztätigkeit eines Patienten um einen Fibrillationszustand handelt und dass es sich bei dem die Störung der Herzaktivität charakterisierenden Parameter um einen Fibrillationsparameter handelt.

3. Verfahren nach Anspruch 1 oder 2, **dadurch gekennzeichnet, dass** eine messtechnische Erfassung eines EKG-Signals, eines Puls-Signals und/oder eines Hämodynamik-Signals durchgeführt wird.

4. Verfahren nach einem der vorhergehenden Ansprüche, **dadurch gekennzeichnet, dass** die Messwerterfassung im Bereich mindestens eines Klebepflasters, Armbandes, Halsbandes, Brustbandes, Bauchbandes, Hüftbandes und/oder im Bereich einer Beatmungsmaske durchgeführt wird.

5. Verfahren nach einem der vorhergehenden Ansprüche, **dadurch gekennzeichnet, dass** eine sensorische Messdatenerfassung und eine Auswertung der Messsignale voneinander örtlich getrennt durchgeführt werden.

6. Verfahren nach einem der Ansprüche 1 bis 4, **dadurch gekennzeichnet, dass** eine sensorische Messdatenerfassung und eine Auswertung der Messsignale örtlich benachbart durchgeführt werden und dass die Ergebnisse der Signalauswertung an einen anderen Ort übertragen werden.

7. Verfahren nach einem der vorhergehenden Ansprüche, **dadurch gekennzeichnet, dass** die vom Sensor (12) erfaßten Messdaten drahtlos zu einer Signalauswertung (13) oder dass die Ergebnisse der Signalauswertung (13) drahtlos zu einem Signalgenerator (14) übertragen werden.

8. Verfahren nach einem der vorhergehenden Ansprüche, **dadurch gekennzeichnet, dass** ein akustisches und/oder ein optisches Alarmsignal generiert wird.

9. Verfahren nach einem der vorhergehenden Ansprüche, **dadurch gekennzeichnet, dass** das Alarmsignal ein Steuersignal umfasst, das unmittelbar die Aktivierung eines Defibrillators bewirkt.

10. Verfahren nach einem der vorhergehenden Ansprüche, **dadurch gekennzeichnet, dass** die Werte des mindestens einen die Herztätigkeit eines Patienten charakterisierenden Parameters gespeichert werden.

11. Verfahren nach einem der vorhergehenden Ansprüche, **dadurch gekennzeichnet, dass** bei Erkennen einer Grenzwertüberschreitung ein Kennzeichnungssignal generiert wird, das die Abgabe des Alarmsignals bewirkt.

12. Verfahren nach Anspruch 11, **dadurch gekennzeichnet, dass** das Kennzeichnungssignal schnurgebunden oder drahtlos übertragen wird.

13. Verfahren nach Anspruch 12, **dadurch gekennzeichnet, dass** die Übertragung des Kennzeichnungssignals per Datennahübertragung, insbesondere per Bluetooth, oder per Datenfernübertragung, insbesondere mittels Telefon oder Mobilfunktechnik, erfolgt.

14. Verfahren nach einem der Ansprüche 11 bis 13, **dadurch gekennzeichnet, dass** im Rahmen der Übertragung des Kennzeichnungssignals die gespeicherten Werte des mindestens einen die Herztätigkeit eines Patienten charakterisierenden Parameters oder Informationen über einen Speicherplatz übertragen werden, an dem die Werte abrufbar vorliegen.

15. Verfahren nach einem der Ansprüche 11 bis 14, **dadurch gekennzeichnet, dass** im Rahmen der Übertragung des Kennzeichnungssignals Patientendaten oder Informationen über einen Speicherplatz, an dem Patientendaten abrufbar vorliegen, übertragen werden.

16. Verfahren nach einem der vorhergehenden Ansprüche, **dadurch gekennzeichnet, dass** erfasst wird, ob und wie sich der Patient bewegt, und dass diese Informationen zusammen mit den die Herztätigkeit eines Patienten charakterisierenden Parametern zur Bestimmung einer Grenzwertüberschreitung herangezogen werden.

17. Vorrichtung zur Erfassung einer Störung der Herztätigkeit eines Patienten mit mindestens einem Sensor (12) zur Erfassung mindestens eines eine Herztätigkeit eines Patienten charakterisierenden Signals, mit wenigstens einer Signalauswertung (13), an die der Sensor (12) angeschlossen ist, wobei die Signalauswertung (13) mit einem Analysator zur Erkennung einer Grenzwertüberschreitung für mindestens einen die Störung der Herztätigkeit charakterisierenden Parameter versehen ist, sowie mit einem Signalgeber (15), an den die Signalauswertung (13) angeschlossen ist.

18. Vorrichtung nach Anspruch 17, **dadurch gekennzeichnet, dass** es sich bei der Störung der Herztätigkeit eines Patienten um einen Fibrillationszustand handelt und dass es sich bei dem die Störung der Herzaktivität charakterisierenden Parameter um einen Fibrillationsparameter handelt.

19. Vorrichtung nach Anspruch 17 oder 18, **dadurch gekennzeichnet, dass** der Signalgeber (15) von einem Signalgenerator (14) aktivierbar ist.

20. Vorrichtung nach einem der vorhergehenden Ansprüche, **dadurch gekennzeichnet, dass** die Vorrichtung als eine mobile Einheit ausgebildet ist und zur Defibrillation dient und ferner einen Spannungsgenerator, eine Steuereinheit (9) sowie mindestens zwei Elektroden (2, 3) aufweist.

21. Vorrichtung nach Anspruch 20, **dadurch gekennzeichnet, dass** die Signalauswertung (13) als Teil der Steuereinheit (9) ausgebildet ist.

22. Vorrichtung nach Anspruch 20 oder 21, **dadurch gekennzeichnet, dass** die Signalauswertung (13) örtlich getrennt zur Steuereinheit (9) angeordnet ist.

23. Vorrichtung nach einem der vorhergeheden Ansprüche, **dadurch gekennzeichnet, dass** der Sensor (12) benachbart oder örtlich getrennt zur Signalauswertung (13) angeordnet ist.

24. Vorrichtung nach einem der vorhergehenden Ansprüche, **dadurch gekennzeichnet, dass** der Sensor (12) drahtlos mit der Signalauswertung (13) gekoppelt ist.

25. Vorrichtung nach einem der vorhergehenden Ansprüche, **dadurch gekennzeichnet, dass** ein Speicher vorgesehen ist, in dem die Werte des mindestens einen die Herztätigkeit eines Patienten charakterisierenden Parameters und/oder Patientendaten gespeichert werden.

26. Vorrichtung nach einem der vorhergehenden Ansprüche, **dadurch gekennzeichnet, dass** der Signalgeber (15) schnurgebunden oder drahtlos mit dem Signalgenerator (14) in Verbindung steht.

27. Vorrichtung nach einem der vorhergehenden Ansprüche, **dadurch gekennzeichnet, dass** Bewegungssensoren vorgesehen sind, mittels derer erfasst wird, ob und wie sich der Patient bewegt.

28. Vorrichtung nach einem der vorhergehenden Ansprüche, **dadurch gekennzeichnet, dass** die Sensoren (12) zur Erfassung mindestens eines eine Herztätigkeit eines Patienten charakterisierendes Signals durch Defibrillatorelektroden gebildet werden.

29. Vorrichtung nach einem der vorhergehenden Ansprüche, **dadurch gekennzeichnet, dass** Mittel vorgesehen sind, anhand derer Informationen zu dem Aufenthaltsort des Patienten erhältlich sind.
